# EUROPEAN PATENT APPLICATION

(11) **EP 4 295 893 A1**
(43) Date of publication of application: **27.12.2023**
(21) Application number: 22755759.2
(22) Date of filing: 11.01.2022
(51) Int. Cl.: A61M 37/00

(54) **MEDICAL DEVICE AND LIGHT-QUANTITY ADJUSTING DEVICE**

(30) Priority: 19.02.2021 JP 2021025317
(71) Applicant: FURUKAWA ELECTRIC CO., LTD., Chiyoda-ku Tokyo 100-8322 (JP)
(72) Inventor: SUEMATSU Katsuki, Tokyo 100-8322 (JP); YAGI Takeshi, Tokyo 100-8322 (JP); HIMURA Atsushi, Tokyo 100-8322 (JP); NARA Kazutaka, Tokyo 100-8322 (JP); ARAI Tsunenori, Tokyo 100-8322 (JP)
(74) Representative: Winter, Brandl - Partnerschaft mbB
(86) International application number: PCT/JP2022/000502
(87) International publication number: WO 2022/176433

(57) **Abstract**

Provided is a medical device including: an extracorporeal unit including a power transmitting unit that transmits electric power from outside of a living body into the living body in a non-contact manner; a medical instrument embedded in the living body, the medical instrument including a power receiving unit that receives the electric power transmitted from the power transmitting unit, a soft portion through which an injection needle is inserted, and a plurality of light emitting units that are provided along an outer edge of the soft portion and are each configured to emit light using the electric power received by the power receiving unit; a light amount adjusting unit that adjusts a light amount of a light-irradiated region including one or more light-irradiated portions to be projected on a body surface from the plurality of light emitting units.

## Description

### TECHNICAL FIELD

The present disclosure relates to a medical device and a light amount adjusting device.

### BACKGROUND ART

In a medical device, in order to inject a medicinal solution into a living body, a body-implantable medical instrument having a main body portion embedded in the living body is used. This medical instrument reduces the burden on a patient who has to be injected frequently for the injection of the medicinal solution. The medical instrument includes a soft portion through which an injection needle is inserted into the main body portion. The soft portion is made of, for example, silicone rubber. Then, in the medical instrument, the medicinal solution is injected into a medicinal solution container through the soft portion. The medicinal solution is transported to the blood vessel through a catheter.

In such a medical instrument, since the main body portion of the device is embedded in the living body, it is difficult to identify the position of the soft portion by visual recognition from outside of the living body.

For example, Patent Document 1 discloses a medical instrument including a light emitting unit. With such a medical instrument, there is a possibility that the position of the soft portion can be confirmed from outside the living body by the light reaching the skin surface which is the body surface.

### PRIOR ART DOCUMENTS

### Patent Documents

Patent Document 1: U.S. Published Patent Application Publication, No. 2004/0199220, Specification

### DISCLOSURE OF THE INVENTION

### Problems to be Solved by the Invention

However, the light emitted from the light emitting unit is absorbed or scattered by the living body tissue. With respect to the light-irradiated portion which is projected on the body surface when the light from the light emitting unit passes through the living body tissue and then reaches the body surface, a decrease in the amount of light due to the living body tissue and an enlargement in the area of the light-irradiated portion occur. Due to the above, a plurality of rays of light emitted from a plurality of light emitting units arranged around the soft portion may overlap with each other, and a single light-irradiated portion may be projected on the body surface instead of a plurality of light-irradiated portions. Therefore, it is difficult to accurately confirm the position of the soft portion in the living body based on the light-irradiated portion on the body surface.

Furthermore, the appearance of the light projected on the body surface varies depending on the state of the patient such as sex and the thickness of the subcutaneous tissue, the environment such as the brightness of the operating room and the treatment room, the state of the user such as the physical condition and the color vision characteristic of the user, and the like.

It is an object of the present disclosure to provide a medical device and a light amount adjusting device which make it possible to easily confirm the position of a soft portion of a medical instrument embedded in a living body from outside of the living body with high accuracy.

### Means for Solving the Problems

[1] A medical device including: an extracorporeal unit including a power transmitting unit that transmits electric power from outside of a living body into the living body in a non-contact manner; a medical instrument embedded in the living body, the medical instrument including a power receiving unit that receives the electric power transmitted from the power transmitting unit, a soft portion through which an injection needle is inserted, and a plurality of light emitting units that are provided along an outer edge of the soft portion and are each configured to emit light using the electric power received by the power receiving unit; and a light amount adjusting unit that adjusts a light amount of a light-irradiated region including one or more light-irradiated portions to be projected on a body surface from the plurality of light emitting units.
[2] The medical device according to [1], in which the light amount adjusting unit adjusts a light amount of the light-irradiated region so that a center of the light-irradiated region is darkened.
[3] The medical device according to [1] or [2], in which the light amount adjusting unit performs adjusting by weakening the light amount of the light-irradiated region.
[4] The medical device according to any one of [1] to [3], in which the light amount adjusting unit simultaneously adjusts light amounts of the plurality of light emitting units.
[5] The medical device according to any one of [1] to [4], further including a stop unit that stops the light amount adjusting unit.
[6] A light amount adjusting device including: an extracorporeal unit including a power transmitting unit that transmits electric power from outside of a living body to a medical instrument embedded in the living body in a non-contact manner, the medical instrument including a plurality of light emitting units that are each configured to emit light, and a light amount adjusting unit that adjusts a light amount of a light-irradiated region including one or more light-irradiated portions to be projected on a body surface from the plurality of light emitting units.

### Effects of the Invention

According to the present disclosure, it is possible to provide a medical device and a light amount adjusting device which make it possible to easily confirm the position of a soft portion of a medical instrument embedded in a living body from outside of the living body with high accuracy.

### BRIEF DESCRIPTION OF THE DRAWINGS

FIG. 1 is a schematic diagram showing an example of a medical device and a light amount adjusting device according to a first embodiment.
FIG. 2 is a block diagram showing a functional configuration of the medical device and the light amount adjusting device according to the first embodiment.
FIG. 3 is a schematic diagram showing an example of an extracorporeal unit constituting the medical device of the first embodiment.
FIG. 4 is a schematic diagram showing an example of a medical instrument constituting the medical device of the first embodiment.
FIG. 5 is a schematic view showing an example of a light-irradiated region projected on a body surface.
FIG. 6 is a schematic view showing another example of a light-irradiated region projected on a body surface.
FIG. 7 is a schematic diagram showing an example of a state of light-irradiated portions and a light-irradiated region projected on a body surface by a medical instrument including two light emitting units, and a relationship of light amounts of the light-irradiated portions and the light-irradiated region on the body surface.
FIG. 8 is a schematic diagram showing another example of the state of the light-irradiated portions and the light-irradiated region projected on the body surface by the medical instrument including the two light emitting units, and the relationship of the light amounts of the light-irradiated portions and the light-irradiated region on the body surface.
FIG. 9 is a schematic diagram showing yet another example of the state of the light-irradiated portions and the light-irradiated region projected on the body surface by the medical instrument including the two light emitting units, and the relationship of the light amounts of the light-irradiated portions and the light-irradiated region on the body surface.
FIG. 10 is a schematic diagram showing an example of a medical device and a light amount adjusting device according to a second embodiment.
FIG. 11 is a block diagram showing a functional configuration of the medical device and the light amount adjusting device according to the second embodiment.
FIG. 12 is a schematic diagram showing an example of a medical device and a light amount adjusting device according to a third embodiment.
FIG. 13 is a block diagram showing a functional configuration of the medical device and the light amount adjusting device according to the third embodiment.
FIG. 14 is a schematic diagram showing an example of a medical device and a light amount adjusting device according to a fourth embodiment.
FIG. 15 is a block diagram showing a functional configuration of the medical device and the light amount adjusting device according to the fourth embodiment.
FIG. 16 is a schematic diagram showing an example of a medical device and a light amount adjusting device according to a fifth embodiment.
FIG. 17 is a block diagram showing a functional configuration of the medical device and the light amount adjusting device according to the fifth embodiment.
FIG. 18 is a schematic diagram showing an example of a medical device and a light amount adjusting device according to a sixth embodiment.
FIG. 19 is a block diagram showing a functional configuration of the medical device and the light amount adjusting device according to the sixth embodiment.
FIG. 20 is a schematic diagram showing an example of a medical instrument constituting the medical device of the sixth embodiment.

### PREFERRED MODE FOR CARRYING OUT THE INVENTION

Hereinafter, embodiments of the present invention will be described in detail.

Medical devices 1, 2, 3, 4, 5, and 6 of the embodiments each include an extracorporeal unit 10 including a power transmitting unit 11 for transmitting electric power from outside of a living body 100 into the living body 100 in a non-contact manner, a medical instrument 20 embedded in the living body 100, the medical instrument 20 including a power receiving unit 25 and 25a for receiving the electric power transmitted from the power transmitting unit 11, a soft portion 23 through which an injection needle 200 is inserted, and a plurality of light emitting units 26 that are provided along an outer edge of the soft portion 23 and are each configured to emit light using the electric power received by the power receiving unit 25 and 25a, and a light amount adjusting unit 30 and 30a for adjusting a light amount of a light-irradiated region including one or more light-irradiated portions 102 to be projected on a body surface 101 from the plurality of light emitting units 26.

Light amount adjusting devices 41, 42, 43, 44, 45 and 46 of the embodiments each include an extracorporeal unit 10 including a power transmitting unit 11 for transmitting electric power from outside of the living body 100 to a medical instrument 20 embedded in the living body 100 in a non-contact manner, the medical instrument 20 including a plurality of light emitting units 26 that are each configured to emit light, and a light amount adjusting unit 30 and 30a for adjusting a light amount of a light-irradiated region including one or more light-irradiated portions 102 to be projected on a body surface 101 from the plurality of light emitting units 26.

### (First Embodiment)

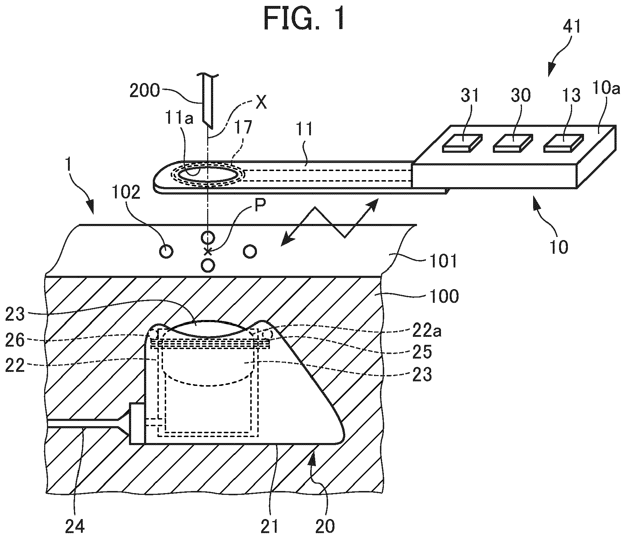

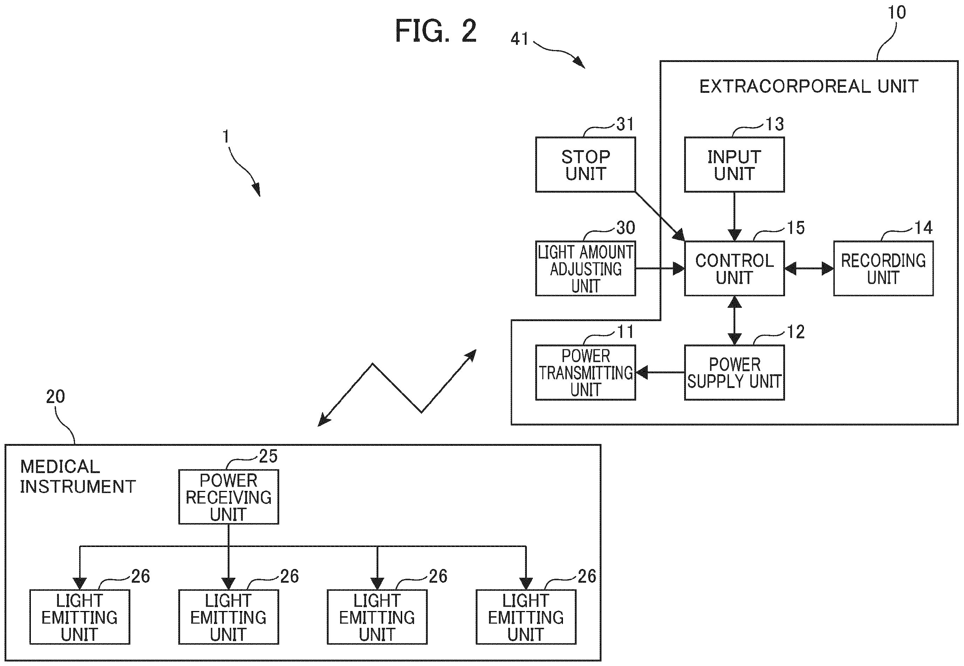

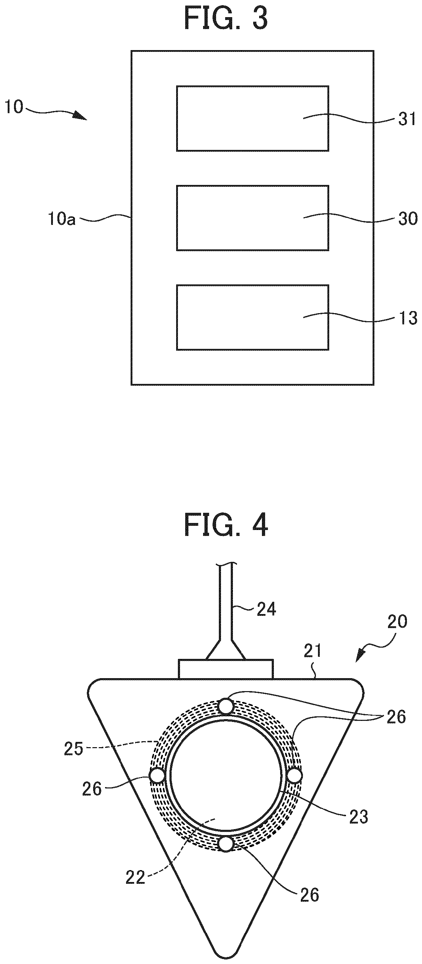

FIG. 1 is a schematic diagram showing an example of a medical device and a light amount adjusting device according to the first embodiment. FIG. 2 is a block diagram showing a functional configuration of the medical device and the light amount adjusting device. FIG. 3 is a schematic diagram showing an example of an extracorporeal unit constituting the medical device. FIG. 4 is a schematic diagram showing an example of a medical instrument constituting the medical device. As shown in FIGS. 1 to 4, the medical device 1 includes the extracorporeal unit 10, the medical instrument 20, and the light amount adjusting unit 30.

As shown in FIG. 1, the extracorporeal unit 10 detects the soft portion 23 of the medical instrument 20 for use in a living body 100 such as a patient or a subject from outside of the living body 100. The extracorporeal unit 10 includes the power transmitting unit 11. The power transmitting unit 11 of the extracorporeal unit 10 transmits electric power from outside of the living body 100 into the living body 100 in a non-contact manner.

As shown in FIG. 1, the medical instrument 20 is embedded in the living body 100. The medical instrument 20 is, for example, referred to as a subcutaneous implantable port (CV port).

As shown in FIGS. 1 to 2, and 4, the medical instrument 20 includes the main body portion 21, the medicinal solution container 22, the soft portion 23, a catheter 24, the power receiving unit 25, and the light emitting unit 26.

The main body portion 21 constituting the medical instrument 20 is a housing made of epoxy resin or the like. The main body portion 21 includes the medicinal solution container 22, the soft portion 23, the catheter 24, the power receiving unit 25 and the light emitting unit 26.

The medicinal solution container 22 is a chamber for temporarily storing the medicinal solution. The medicinal solution container 22 has a circular opening 22a toward the outside of the living body.

The soft portion 23 is referred to as a septum, and closes the opening 22a of the medicinal solution container 22. The soft portion 23 is a soft lid (silicone diaphragm) made of, for example, silicone rubber, and is exposed from the main body portion 21. The soft portion 23 is a portion through which the injection needle 200 for injecting a medicinal solution (transfusion) punctured from the body surface 101 of the living body 100 is inserted. For example, the soft portion 23 has a cylindrical shape.

One end of the catheter 24 communicates with the medicinal solution container 22, and the other end of the catheter 24 communicates with a blood vessel (not shown). The catheter 24 transports the medicinal solution in the medicinal solution container 22 to a blood vessel or the like.

The power receiving unit 25 receives the electric power transmitted from the power transmitting unit 11 of the extracorporeal unit 10. For example, as shown in FIGS. 1 and 4, the power receiving unit 25 includes a coil with annular shape wound a plurality of times around an end face of the medicinal solution container 22, the end face being located toward the outside of the living body, and the power receiving unit 25 is provided around the soft portion 23. The coil constituting the power receiving unit 25 may be made of a wire material made of a copper-based material such as pure copper in order to reduce size and increase power supply, or may be made of a wire material made of an aluminum-based material such as pure aluminum in order to reduce weight. For example, the power receiving unit 25 is integrated with the main body portion 21 by molding or the like.

The plurality of light emitting units 26 are disposed along the outer edge of the soft portion 23, and emit light using the electric power received by the power receiving unit 25 by way of non-contact power supply from the power transmitting unit 11 of the extracorporeal unit 10. The light irradiated from the plurality of light emitting units 26 passes through the living body tissue to reach the body surface 101, and projects a light-irradiated region including one or more light-irradiated portions 102 on the body surface 101. The amount of light in the light-irradiated portion 102 is not uniform. For example, the amount of light in the light-irradiated portion 102 is the largest at the center of the light-irradiated portion 102, and decreases as the distance from the center increases. In addition, the amount of light in the light-irradiated region is also not uniform.

The plurality of light emitting units 26 are arranged in an annular manner around the soft portion 23 at predetermined intervals. Here, the four light emitting units 26 are arranged in an annular manner around the soft portion 23 at equal intervals. The arrangement conditions of the plurality of light emitting units 26 such as the number and the position of the plurality of light emitting units 26 is appropriately set.

The plurality of light emitting units 26 emit light according to the magnitude of the electric power received by the power receiving unit 25. As the electric power received by the power receiving unit 25 is larger, the amount of the irradiation light irradiated from the light emitting unit 26 is larger. The light amounts of the light emitting units 26 are the same, for example. The plurality of light emitting units 26 are preferably LEDs having high directivity, and among them, a red LED that emits light of the red wavelength band is more preferable from the viewpoint of good transmission in the living body 100.

As shown in FIG. 1, one end of a plate-shaped power transmitting unit 11 is coupled with a housing 10a of the extracorporeal unit 10. At the other end side of the power transmitting unit 11, a through hole 11a penetrating from one main surface to the other main surface of the power transmitting unit 11 is provided. The size of the through hole 11a is larger than the outer dimension of the injection needle 200. The through hole 11a has, for example, a circular shape.

The power transmitting unit 11 includes a power transmitting coil 17 therein. The power transmitting coil 17 provided inside the power transmitting unit 11 is wound a plurality of times around the outside of the through hole 11a in an annular shape, and is connected to the power supply unit 12 shown in FIG. 2.

As shown in FIG. 2, the housing 10a of the extracorporeal unit 10 houses the power supply unit 12, an input unit 13, a recording unit 14, and a control unit 15 (an extracorporeal unit-side control unit).

The power supply unit 12 of the extracorporeal unit 10 supplies electric power to the power transmitting unit 11 under the control of the control unit 15. The power supply unit 12 includes a battery, a booster circuit, and the like.

The input unit 13 is operated by a user's input operation. For example, the input unit 13 includes buttons, switches, and a touch screen. The user may be a doctor, a nurse, a caregiver, a patient, a subject, or the like.

The recording unit 14 records various programs and information executed by the extracorporeal unit 10. The recording unit 14 includes volatile memory, non-volatile memory, and the like.

The control unit 15 controls the power supply unit 12 and the recording unit 14 of the extracorporeal unit 10. When the input unit 13 is pressed, the control unit 15 controls the power supply unit 12 to supply the electric power from the power supply unit 12 to the power transmitting unit 11. The control unit 15 includes memory and a processor having hardware such as a CPU (Central Processing Unit).

The power transmitting unit 11 transmits the electric power supplied from the power supply unit 12 to the power receiving unit 25 in the living body 100 in a non-contact manner. For example, as shown in FIG. 1, the power transmitting coil 17 in the power transmitting unit 11 connected to the power supply unit 12 transmits the electric power supplied from the power supply unit 12 to the power receiving unit 25 of the medical instrument 20 in a non-contact manner. Furthermore, for example, even when the power transmitting unit 11 and the power receiving unit 25 are separated by several hundreds of millimeters, the electric power transmitted from the power transmitting unit 11 to the power receiving unit 25 allows the light emitting unit 26 to emit light.

The light amount adjusting unit 30 of the medical device 1 adjusts the light amount of the light-irradiated region including one or more light-irradiated portions 102 projected on the body surface 101 from the plurality of light emitting units 26. The light amount adjusting unit 30 is located outside the living body 100. As shown in FIG. 1, the light amount adjusting unit 30 is integrated with the housing 10a of the extracorporeal unit 10. Alternatively, the light amount adjusting unit 30 may be separated from the extracorporeal unit 10.

The light amount adjusting unit 30 is operated by a user. For example, the light amount adjusting unit 30 includes a button, a switch, a touch screen, and the like.

When the user adjusts the light amount adjusting unit 30, the control unit 15 controls the power supply unit 12 to adjust the magnitude of the electric power supplied from the power supply unit 12 to the power transmitting unit 11. When the magnitude of the electric power supplied to the power transmitting unit 11 is adjusted, the magnitude of the electric power supplied to the plurality of light emitting units 26 via the power receiving unit 25 is adjusted, so that the light amount adjusting unit 30 can simultaneously adjust the amount of light of the plurality of light emitting units 26, in this case, the amount of light of all the light emitting units 26. In this way, since the light amount adjusting unit 30 changes the magnitude of the electric power supplied to the light emitting unit 26, it is possible for the medical device 1 to adjust the light-irradiated region projected on the body surface 101 from the light emitting unit 26.

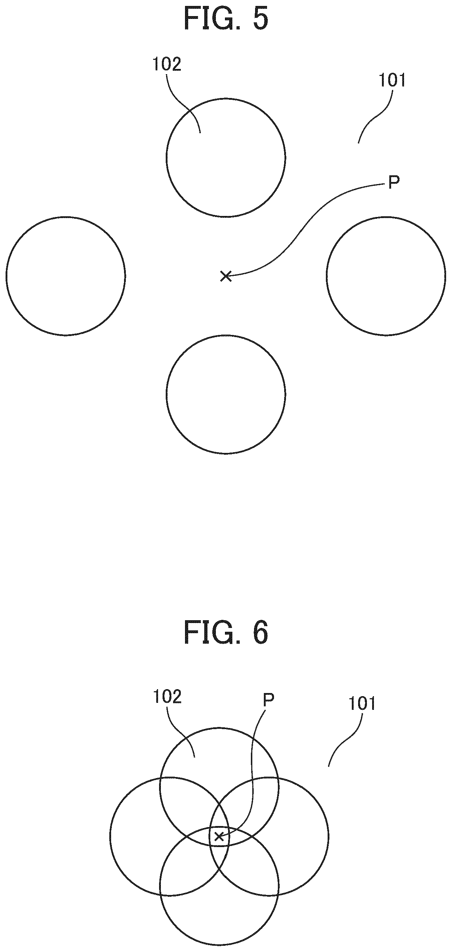

FIGS. 5 and 6 are schematic views each showing a light-irradiated region projected on the body surface 101. For example, as shown in FIG. 5, when a plurality of light-irradiated portions 102 are projected on the body surface 101, the puncture target area P that is located at the center of the light-irradiated region and where the injection needle 200 punctures on the body surface 101 is an area where the light of the light emitting units 26 is not reached, that is, an area where the light of the light emitting units 26 is not irradiated. In addition, as shown in FIG. 6, when one light-irradiated portion 102 is projected on the body surface 101, the puncture target area P in the light-irradiated region is an area where the light of the light emitting unit 26 is irradiated. As shown in FIGS. 5 and 6, the state in which the plurality of light-irradiated portions 102 are projected on the body surface 101 and the state in which one light-irradiated portion 102 is projected on the body surface 101 depend on the amount of the light irradiated from the light emitting unit 26.

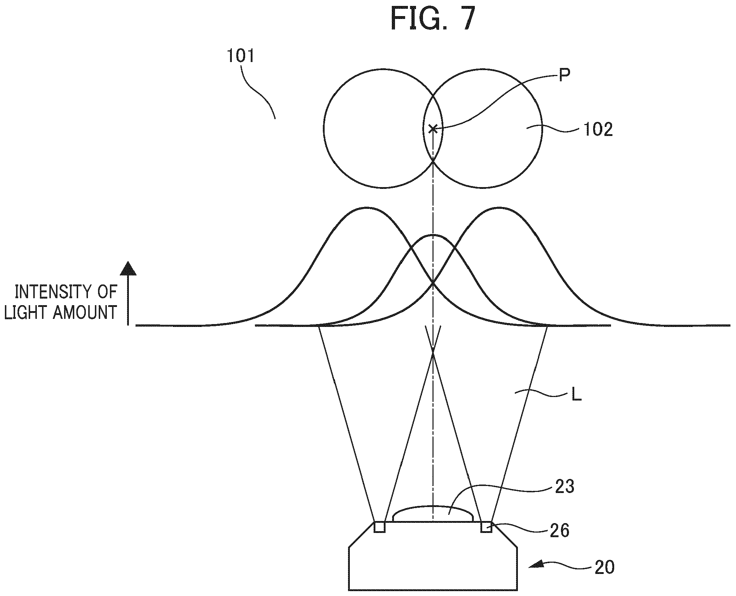

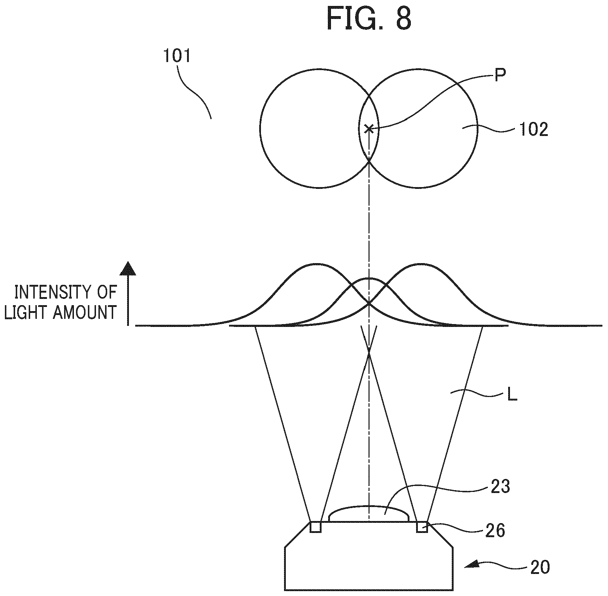

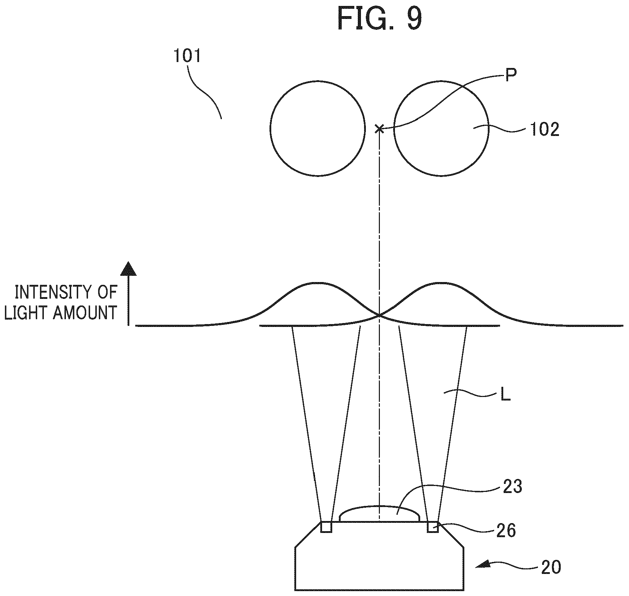

FIGS. 7 to 9 are schematic views each showing the state of the light-irradiated portions 102 and the light-irradiated region projected on the body surface 101 projected by the medical instrument 20 including the two light emitting units 26, and the relationship of the light amounts of the light-irradiated portions 102 and the light-irradiated region on the body surface 101. FIG. 7 shows a state in which the amount of light on the body surface 101 is large, FIG. 8 shows a state in which the amount of light is smaller than that in FIG. 7, and FIG. 9 shows a state in which the amount of light is smaller than that in FIG. 8. In each of FIGS. 7 to 9, an example of a medical instrument including two light emitting units will be described in order to explain in a simple manner the state of the light-irradiated portions 102 and the light-irradiated region on the body surface 101, and the light amounts of the light-irradiated portions 102 and the light-irradiated region on the body surface 101.

As shown in FIG. 7, in a state where the amount of light on the body surface 101 is large, one light-irradiated portion 102 as shown in FIG. 6 is projected on the body surface 101. In this state, at the puncture target area P that indicates the position of the center of the soft portion 23 and is located in the light-irradiated region, the light L irradiated from the plurality of light emitting units 26 overlap. When the amount of light is gradually reduced from the state shown in FIG. 7, the amount of light of the light-irradiated portions 102 and the amount of light of the puncture target area P decrease while maintaining the state in which the light L irradiated from the plurality of light emitting units 26 overlap at the puncture target area P, as shown in FIG. 8. As shown in FIG. 9, when the amount of light is further reduced, the plurality of light-irradiated portions 102 as shown in FIG. 5 are projected on the body surface 101. In this state, the light L irradiated from the plurality of light emitting units 26 does not reach the puncture target area P.

The user can adjust the light amount adjusting unit 30 while checking the state of the light-irradiated region projected on the body surface 101, and can adjust the state of the light-irradiated region by simultaneously adjusting the light amounts of all the light emitting units 26. Then, the user can check the soft portion 23 of the medical instrument 20 by adjusting the state of the light-irradiated region, in consideration of the light amounts of the light-irradiated portions 102 and the light-irradiated region on the body surface 101, the state of the puncture target area P indicating the position of the center of the soft portion 23, and the like.

Next, a procedure for checking the position of the soft portion 23 of the medical instrument 20 embedded in the living body 100 will be described.

The user presses the input unit 13 of the extracorporeal unit 10 to supply the electric power from the power supply unit 12 to the power transmitting unit 11. In this state, the user scans the extracorporeal unit 10 while approaching the body surface 101. When the power transmitting unit 11 of the extracorporeal unit 10 approaches the power receiving unit 25 of the medical instrument 20, the power receiving unit 25 receives the electric power from the power transmitting unit 11 in a non-contact manner. The plurality of light emitting units 26 emit light by the electric power received by the power receiving unit 25. The light amount of the plurality of light emitting units 26 depends on the magnitude of the electric power received by the power receiving unit 25. The user can adjust the magnitude of the electric power received by the power receiving unit 25 and adjust the state of the light-irradiated region by adjusting the light amount adjusting unit 30 while checking the state of the light-irradiated region projected on the body surface 101 by the plurality of light emitting units 26. In this way, it is possible for the user to easily check the position of the soft portion 23 of the medical instrument 20 embedded in the living body 100 from outside of the living body 100 with high accuracy by adjusting the state of the light-irradiated region using the light amount adjusting unit 30. Furthermore, since the light amounts of all the light emitting units 26 can be adjusted at the same time, it is possible to easily adjust the state of the light-irradiated region.

After checking the position of the soft portion 23 in the living body 100 from outside of the living body 100, the user inserts the injection needle 200 into the through hole 11a of the power transmitting unit 11 and then punctures the injection needle 200 at the puncture target area P of the body surface 101 to inserts the injection needle 200 into the soft portion 23. In this way, since the injection needle 200 can be easily inserted into the soft portion 23 which is easily confirmed from outside of the living body 100 with high accuracy, it is possible to easily inject the medicinal solution from the injection needle 200 into the medicinal solution container 22.

The light amount adjusting unit 30 preferably adjusts the light amount of the light-irradiated region so that the center of the light-irradiated region is darkened. As described above, the center of the light-irradiated region projected on the body surface 101 is the puncture target area P.

As shown in FIGS. 7 and 8, when the light amount of the light emitting unit 26 is large, the contrast of light and darkness may be low at the puncture target area P indicating the position of the center of the soft portion 23 and in the portion of the light-irradiated portion 102 excluding the puncture target area P (hereinafter, also referred to as a portion of the light-irradiated portion 102 other than the puncture target area P). In contrast, as shown in FIG. 9, when the light amount of the light emitting unit 26 is reduced, the contrast of the light and darkness at the puncture target area P and in the portion of the light-irradiated portion 102 is improved.

When the light amount of the light-irradiated region is adjusted by the light amount adjusting unit 30 so as to decrease the light amount of the puncture target area P from the state where the light of the light emitting unit 26 reaches the puncture target area P in the light-irradiated region as shown in FIGS. 7 and 8, a result of which the light of the light emitting unit 26 does not reach the puncture target area P as shown in FIG. 9, it is possible to easily discriminate the puncture target area P based on the light-irradiated region projected on the body surface 101. Therefore, it is possible to easily confirm the position of the soft portion 23 in the living body 100 from outside of the living body 100 with higher accuracy.

Furthermore, by adjusting the light amount of the light-irradiated region by the light amount adjusting unit 30, and making the luminance of the puncture target area P smaller than the luminance of the portion of the light-irradiated portion 102 other than the puncture target area P, and setting the difference between the luminance of the puncture target area P and the luminance of the portion of the light-irradiated portion 102 other than the puncture target area P (the luminance of the portion of the light-irradiated portion 102 other than the puncture target area P - the luminance of the puncture target area P) to 2% or more, the contrast between the puncture target area P and the portion of the light-irradiated portion 102 other than the puncture target area P is further improved, so that it is possible for the user to more easily discriminate the puncture target area P from the light-irradiated region. Therefore, it is possible to confirm the position of the soft portion 23 in the living body 100 from outside of the living body 100 with higher accuracy.

Furthermore, it is preferable that the light amount adjusting unit 30 adjusts the light amount of the light-irradiated region by weakening the light amount. When the light amount of the light-irradiated region is adjusted by weakening the light amount of the light-irradiated region, rather than adjusting by increasing the light amount of the light-irradiated region or adjusting by repeating the increasing and weakening the light amount, the contrast adjustment of the puncture target area P and the portion of the light-irradiated portion 102 other than the puncture target area P, to easily discriminate the puncture target area P based on the light-irradiated region as described above, becomes easier. Furthermore, when the light amount adjusting unit 30 adjusts the light amount of the light-irradiated region by weakening so that the luminance of the puncture target area P is smaller than the luminance of the portion of the light-irradiated portion 102 other than the puncture target area P and the difference between the luminance of the puncture target area P and the luminance of the portion of the light-irradiated portion 102 other than the puncture target area P is sufficiently recognized, for example, 2% or more, it is possible for the user to easily discriminate the puncture target area P from the light-irradiated region by easily adjusting the contrast between the puncture target area P and the portion of the light-irradiated portion 102 other than the puncture target area P. With such a configuration, by adjusting the light amount of the light-irradiated region while weakening the light amount by the light amount adjusting unit 30, it is possible to easily confirm the position of the soft portion 23 with higher accuracy. It is also effective to adjust the light amount of the light-irradiated region for each unit in which the user can easily recognize the change in the contrast adjustment so that the change in the light amount of the light-irradiated region including the light-irradiated portions 102 projected on the body surface 101 can be easily recognized. For example, the amount of electric power supplied from the power transmitting unit 11 of the extracorporeal unit 10 to the power receiving unit 25 of the medical instrument 20 may be changed in a stepwise manner for each unit in which the user can easily recognize the change in contrast.

Preferably, the medical device 1 further includes a stop unit 31 for stopping the light amount adjusting unit 30. As shown in FIG. 1, the stop unit 31 is integrated with the housing 10a of the extracorporeal unit 10. Alternatively, the stop unit 31 may be separated from the extracorporeal unit 10.

The stop unit 31 is operated by a user. For example, the stop unit 31 includes a button, a switch, a foot switch, and a touch screen. The stop unit 31 may be operated by voice input or line-of-sight input.

When the user activates the stop unit 31, the control unit 15 controls the light amount adjusting unit 30 to stop the adjustment of the light amount of the light-irradiated region by the light amount adjusting unit 30. The user stops the light amount adjusting unit 30 by the stop unit 31 to maintain the state of the light-irradiated region in which the puncture target area P can be easily discriminated from the light-irradiated region, when the state of the light-irradiated region in which the puncture target area P of the light-irradiated region can be easily discriminated is established while adjusting the light amount of the light-irradiated region by the light amount adjusting unit 30. Therefore, it is possible to easily confirm the position of the soft portion 23 in the living body 100 from outside of the living body 100 with higher accuracy.

The light amount adjusting device 41 includes the extracorporeal unit 10 and the light amount adjusting unit 30. Since it is possible for the light amount adjusting device 41 to easily confirm the position of the soft portion 23 of the medical instrument 20 embedded in the living body 100 from outside of the living body 100 with high accuracy by the extracorporeal unit 10 and the light amount adjusting unit 30, it is possible to easily inject the medicinal solution from the injection needle 200 into the medicinal solution container 22.

Furthermore, it is preferable that the light amount adjusting device 41 includes the stop unit 31. The stop unit 31 stops the light amount adjusting unit 30. It is possible for the light amount adjusting device 41 to easily confirm the position of the soft portion 23 in the living body 100 from outside of the living body 100 with higher accuracy by the stop unit 31.

According to the first embodiment described above, the extracorporeal unit 10 allows the light emitting unit 26 mounted on the medical instrument 20 in the living body 100 to emit light, and the light amount adjusting unit 30 adjusts the light amount of the light-irradiated region projected on the body surface 101 from the light emitting unit 26, whereby it is possible to easily confirm the position of the soft portion 23 constituting the medical instrument 20 in the living body 100 from outside of the living body 100 with high accuracy.

In the above description, as shown in FIG. 1, an example of the power transmitting unit 11 that is coupled with the housing 10a and includes the through hole 11a and the power transmitting coil 17 having an annular shape therein is shown. However, the configuration of the power transmitting unit 11 is not particularly limited as long as electric power can be transmitted from outside of the living body to the power receiving unit 25 of the medical instrument 20 embedded in the living body in a non-contact manner.

For example, the power transmitting unit 11 may be removably coupled with the housing 10a. In this case, each of the power transmitting unit 11 and the housing 10a includes a connector portion (not shown). By coupling the connector portion of the power transmitting unit 11 with the connector portion of the housing 10a, the power transmitting unit 11 can be coupled to the housing 10a. Furthermore, by detaching the connector portion of the power transmitting unit 11 coupled to the housing 10a from the connector portion of the housing 10a, the power transmitting unit 11 can be detached from the housing 10a.

### (Second Embodiment)

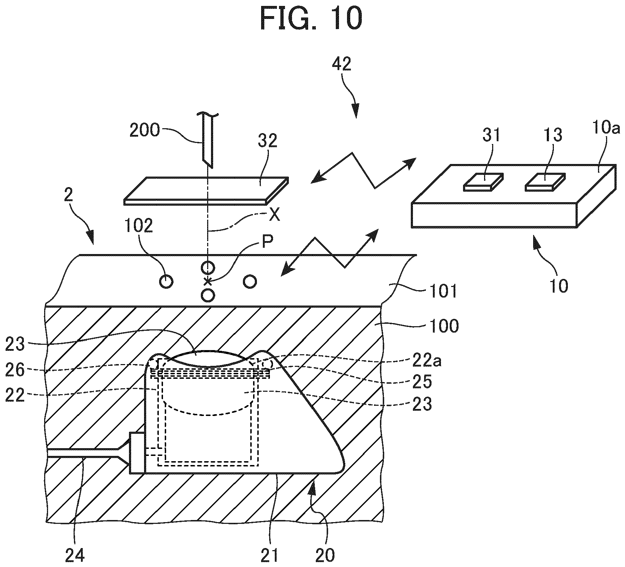

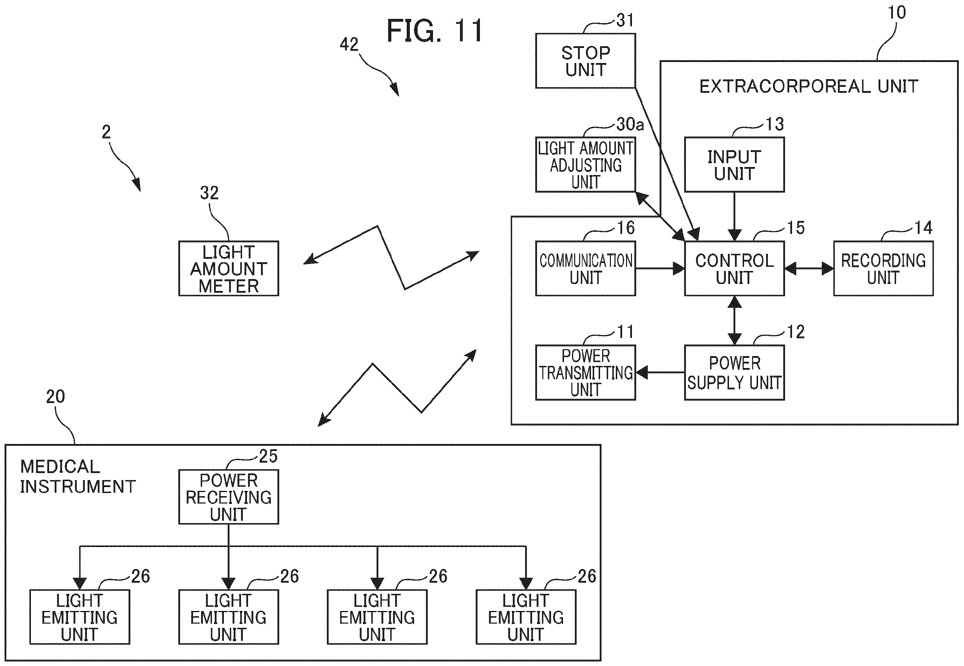

FIG. 10 is a schematic diagram showing an example of a medical device and a light amount adjusting device according to a second embodiment. FIG. 11 is a block diagram showing a functional configuration of the medical device and the light amount adjusting device.

In the following embodiments, the same components as those of the medical device 1 and the light amount adjusting device 41 of the first embodiment are denoted by the same reference numerals, and redundant descriptions thereof will be omitted or simplified.

A medical device 2 and a light amount adjusting device 42 of the second embodiment are basically the same as the medical device 1 and the light amount adjusting device 41 of the first embodiment except that the configuration of a light amount adjusting unit 30a differs from the light amount adjusting unit 30 of the first embodiment and a communication unit 16 (extracorporeal unit-side communication unit) and a light amount meter 32 are added. Therefore, herein, different configurations therebetween will be mainly described.

As shown in FIGS. 10 and 11, the medical device 2 includes an extracorporeal unit 10, a medical instrument 20, a light amount adjusting unit 30a, and a light amount meter 32. The medical device 2 may also include a stop unit 31.

The light amount meter 32 measures the light amount of the light-irradiated region including one or more light-irradiated portions 102 projected on the body surface 101. In addition, the light amount meter 32 transmits a measurement result of the light amount of the light-irradiated region to the communication unit 16 of the extracorporeal unit 10. The light amount meter 32 is located outside of the living body 100.

The light amount meter 32 is separate from the extracorporeal unit 10 as shown in FIG. 10, but may be integrated with the housing 10a of the extracorporeal unit 10. Furthermore, as shown in FIG. 10, the light amount meter 32 may be disposed above the light-irradiated region apart from the body surface 101, or may be disposed on the body surface 101.

In addition to the above configuration, the extracorporeal unit 10 further includes a communication unit 16. Under the control of the control unit 15, the communication unit 16 outputs the measurement result of the light amount of the light-irradiated region received from the light amount meter 32 to the control unit 15.

The extracorporeal unit 10 includes the light amount adjusting unit 30a instead of the light amount adjusting unit 30. The adjustment of the light amount of the light-irradiated region by the light amount adjusting unit 30a is automatically performed by the control unit 15 instead of operation by the user as described above. The control unit 15 adjusts the light amount of the light-irradiated region by adjusting the light amount adjusting unit 30a based on the measurement result of the light amount of the light-irradiated region outputted from the communication unit 16. For example, the control unit 15 refers to biological data such as the body type and the like and the light amount data of the light-irradiated region recorded in the recording unit 14, and adjusts the light amount adjusting unit 30a based on the measurement result of the light amount of the light-irradiated region. Furthermore, the control unit 15 may autonomously learn the optimum value of the light amount of the light-irradiated region based on a plurality of results measured by the light amount meter 32, and a plurality of pieces of biological data and light amount data in the recording unit 14, thereby adjusting the light amount adjusting unit 30a.

In this way, the medical device 2 automatically adjusts the light amount adjusting unit 30a based on the light amount of the light-irradiated region. In this way, since the light amount adjusting unit 30a can be automated, the operation of confirming the soft portion 23 in the living body 100 can be simplified.

The light amount adjusting device 42 includes the extracorporeal unit 10, the light amount adjusting unit 30a, and the light amount meter 32. Furthermore, the light amount adjusting device 42 may include a stop unit 31. The light amount adjusting device 42 automatically adjusts the light amount adjusting unit 30a based on the light amount of the light-irradiated region. In this way, since the light amount adjusting unit 30a can be automated, the operation of confirming the soft portion 23 in the living body 100 can be simplified.

According to the second embodiment described above, since the light amount of the light-irradiated region projected on the body surface 101 is measured by the light amount meter 32, and the light amount adjusting unit 30a can be adjusted by the control unit 15 based on the result of the light amount of the light-irradiated region measured by the light amount meter 32, the light amount adjusting unit 30a can be automated. Therefore, it is easy to confirm the soft portion 23 constituting the medical instrument 20 embedded in the living body 100.

Furthermore, the medical device 2 and the light amount adjusting device 42 may include a camera including an image processing unit instead of the light amount meter 32. Such a camera captures an image of a light-irradiated region including one or more light-irradiated portions 102 projected on the body surface 101, and the image processing unit processes the image of the light-irradiated region obtained by the imaging. Subsequently, the camera transmits the data obtained by the image processing to the communication unit 16.

The communication unit 16 of the extracorporeal unit 10 outputs data received from the camera to the control unit 15. The control unit 15 adjusts the light amount of the light-irradiated region by adjusting the light amount adjusting unit 30a based on the data outputted from the communication unit 16. For example, the control unit 15 adjusts the light amount adjusting unit 30a by referring to the data recorded in the recording unit 14.

### (Third Embodiment)

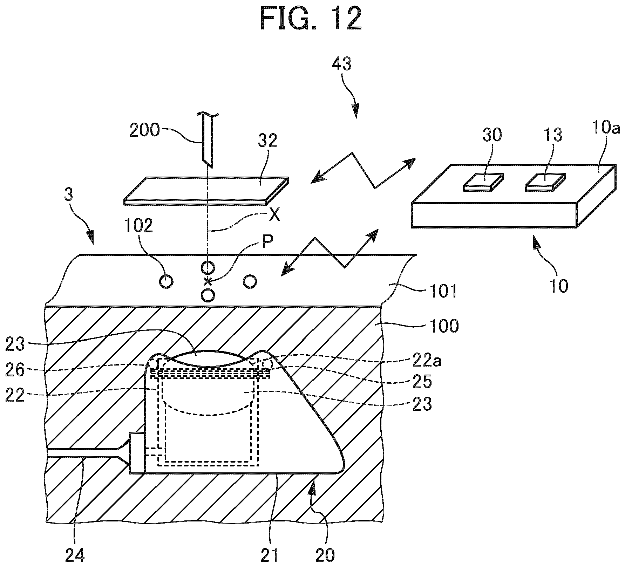

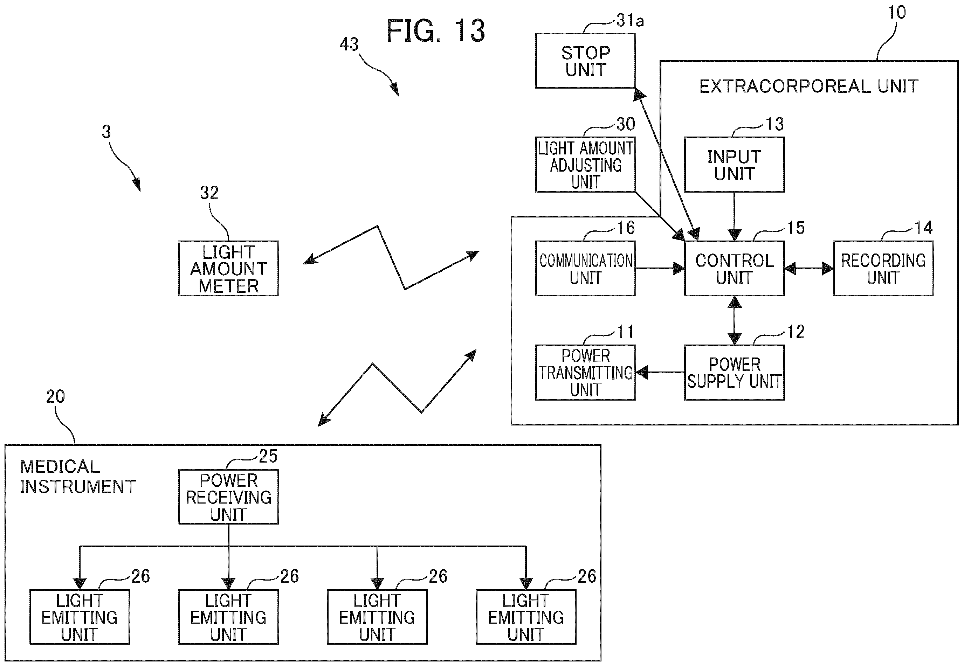

FIG. 12 is a schematic diagram showing an example of a medical device and a light amount adjusting device according to a third embodiment. FIG. 13 is a block diagram showing a functional configuration of the medical device and the light amount adjusting device.

A medical device 3 and a light amount adjusting device 43 of the third embodiment are basically the same as the medical device 1 and the light amount adjusting device 41 of the first embodiment, except that the configuration of a stop unit 31a differs from the stop unit 31, and the communication unit 16 and the light amount meter 32 are added. Therefore, herein, different configurations therebetween will be mainly described.

As shown in FIGS. 12 and 13, the medical device 3 includes an extracorporeal unit 10, a medical instrument 20, a light amount adjusting unit 30, a stop unit 31a, and a light amount meter 32.

The light amount meter 32 measures the light amount of the light-irradiated region including one or more light-irradiated portions 102 projected on the body surface 101. In addition, the light amount meter 32 transmits the measurement result of the light amount of the light-irradiated region to the communication unit 16 of the extracorporeal unit 10. The light amount meter 32 is located outside of the living body 100.

The light amount meter 32 is separate from the extracorporeal unit 10 as shown in FIG. 12, but may be integrated with the housing 10a of the extracorporeal unit 10. Furthermore, as shown in FIG. 12, the light amount meter 32 may be disposed above the light-irradiated region apart from the body surface 101, or may be disposed on the body surface 101.

In addition to the above configuration, the extracorporeal unit 10 further includes a communication unit 16. Under the control of the control unit 15, the communication unit 16 outputs the measurement result of the light amount of the light-irradiated region received from the light amount meter 32 to the control unit 15.

The extracorporeal unit 10 includes a stop unit 31a instead of the stop unit 31. The stop of the light amount adjusting unit 30 by the stop unit 31a is not operated by the user as described above, but is automatically performed by the control unit 15. The control unit 15 activates the stop unit 31a based on the measurement result of the light amount of the light-irradiated region outputted from the communication unit 16, and stops the adjustment of the light amount of the light-irradiated region by the light amount adjusting unit 30. For example, the control unit 15 refers to the biological data and the light amount data of the light-irradiated region recorded in the recording unit 14, and stops the light amount adjusting unit 30 based on the measurement result of the light amount of the light-irradiated region. Furthermore, the control unit 15 may autonomously learn the optimum value of the light amount of the light-irradiated region based on the plurality of results measured by the light amount meter 32, and the plurality of pieces of biological data and light amount data in the recording unit 14, thereby activating the stop unit 31a.

In this way, the medical device 3 automatically activates the stop unit 31a based on the light amount of the light-irradiated region. In this way, since the stop unit 31a can be automated, the operation of confirming the soft portion 23 in the living body 100 becomes simpler.

The light amount adjusting device 43 includes the extracorporeal unit 10, the light amount adjusting unit 30, the stop unit 31a, and the light amount meter 32. The light amount adjusting device 43 automatically activates the stop unit 31a based on the light amount of the light-irradiated region. In this way, since the stop unit 31a can be automated, the operation of confirming the soft portion 23 in the living body 100 becomes simpler.

According to the third embodiment described above, since the light amount of the light-irradiated region projected on the body surface 101 is measured by the light amount meter 32, and the stop unit 31a can be activated by the control unit 15 based on the result of the light amount of the light-irradiated region measured by the light amount meter 32, the stop unit 31a can be automated. Therefore, the operation of confirming the soft portion 23 constituting the medical instrument 20 in the living body 100 becomes simpler.

The medical device 3 and the light amount adjusting device 43 may include the light amount adjusting unit 30a instead of the light amount adjusting unit 30. The automation of the light amount adjusting unit 30a by the medical device 3 and the light amount adjusting device 43 further simplifies the operation of confirming the soft portion 23 constituting the medical instrument 20 in the living body 100.

### (Fourth Embodiment)

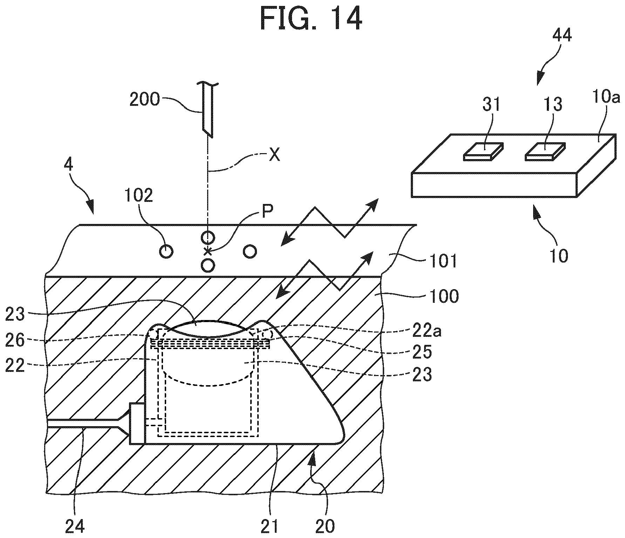

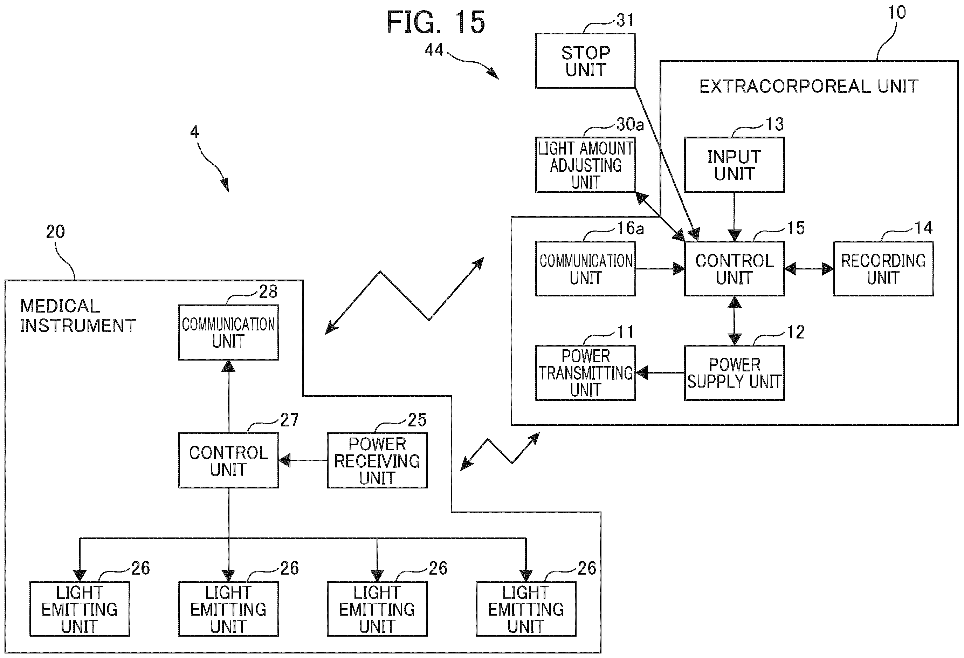

FIG. 14 is a schematic diagram showing an example of a medical device and a light amount adjusting device according to a fourth embodiment. FIG. 15 is a block diagram showing a functional configuration of the medical device and the light amount adjusting device.

A medical device 4 and a light amount adjusting device 44 of the fourth embodiment are basically the same as the medical device 1 and the light amount adjusting device 41 of the first embodiment except that the configuration of the light amount adjusting unit 30a differs from the light amount adjusting unit 30, and that a communication unit 16a, a control unit 27 (medical instrument-side control unit), and a communication unit 28 (medical instrument-side communication unit) are added. Therefore, herein, different configurations therebetween will be mainly described.

As shown in FIGS. 14 and 15, the medical device 4 includes an extracorporeal unit 10, a medical instrument 20, and a light amount adjusting unit 30a. The medical device 4 may also include a stop unit 31. The extracorporeal unit 10 further includes a communication unit 16a. In addition to the above configuration, the medical instrument 20 further includes a control unit 27 and a communication unit 28.

The control unit 27 mounted on the medical instrument 20 in the living body 100 receives the electric power received by the power receiving unit 25 and outputs the result of the magnitude of the received electric power to the communication unit 28. The communication unit 28 transmits the result of the magnitude of the electric power outputted from the control unit 27 to the communication unit 16a of the extracorporeal unit 10. The control unit 27 and the communication unit 28 may be integrally provided in the IC chip or may be separately provided. When a patient of which the medical instrument 20 is embedded in the living body 100 utilizes MRI, the IC chip may be exposed to a strong magnetic field. Therefore, the medical instrument 20 may include an electric current detection function for detecting an electric current flowing through the power receiving unit 25 and a cutoff function such as a switch for electrically disconnecting the power receiving unit 25, the control unit 27, and the communication unit 28. According to this configuration, when the IC chip detects a large electric current equal to or greater than a predetermined threshold value by the electric current detection function, it is possible for the IC chip to electrically disconnect the power receiving unit 25, the control unit 27, and the communication unit 28 by the cutoff function, thereby making it possible to interrupt the electric current flowing through the communication unit 28.

The communication unit 16a of the extracorporeal unit 10 outputs the result of the magnitude of the electric power received from the communication unit 28 of the medical instrument 20 to the control unit 15 under the control of the control unit 15. The control unit 15 adjusts the light amount of the light-irradiated region by adjusting the light amount adjusting unit 30a based on the result of the magnitude of the received electric power of the power receiving unit 25 outputted from the communication unit 16a. For example, the control unit 15 refers to the biological data, the light amount data of the light-irradiated region, and the data of the received electric power of the power receiving unit 25 recorded in the recording unit 14, and adjusts the light amount adjusting unit 30a based on the result of the magnitude of the received electric power of the power receiving unit 25. Furthermore, the control unit 15 may autonomously learn the optimum value of the light amount of the light-irradiated region based on the results of the magnitudes of the plurality of received electric powers measured by the power receiving unit 25 and the plurality of pieces of biological data, the light amount data of the light-irradiated region, and the data of the received electric power of the power receiving unit 25 in the recording unit 14, thereby adjusting the light amount adjusting unit 30a. The light amount adjusting unit 30a automatically adjusts the light amount of the light-irradiated region by the control unit 15.

In this way, the medical device 4 automatically adjusts the light amount adjusting unit 30a based on the magnitude of the electric power received by the power receiving unit 25 constituting the medical instrument 20 in the living body 100. In this way, since the light amount adjusting unit 30a can be automated based on the information in the living body 100 indicating the magnitude of the electric power received by the power receiving unit 25, the operation of confirming the soft portion 23 in the living body 100 becomes simple.

The light amount adjusting device 44 includes the extracorporeal unit 10 and the light amount adjusting unit 30a. Furthermore, the light amount adjusting device 44 may include a stop unit 31. The light amount adjusting device 44 automatically adjusts the light amount adjusting unit 30a based on the magnitude of the electric power received by the power receiving unit 25 constituting the medical instrument 20 in the living body 100. In this way, since the light amount adjusting unit 30a can be automated based on the information in the living body 100 indicating the magnitude of the electric power received by the power receiving unit 25, the operation of confirming the soft portion 23 in the living body 100 becomes simple.

According to the fourth embodiment described above, since the electric power transmitted from the power transmitting unit 11 of the extracorporeal unit 10 to the medical instrument 20 in the living body 100 is received by the power receiving unit 25, and the light amount adjusting unit 30a can be adjusted by the control unit 15 based on the result of the magnitude of the electric power received by the power receiving unit 25 in the living body 100, the light amount adjusting unit 30a can be automated. Therefore, it is easy to confirm the soft portion 23 constituting the medical instrument 20 in the living body 100.

### (Fifth Embodiment)

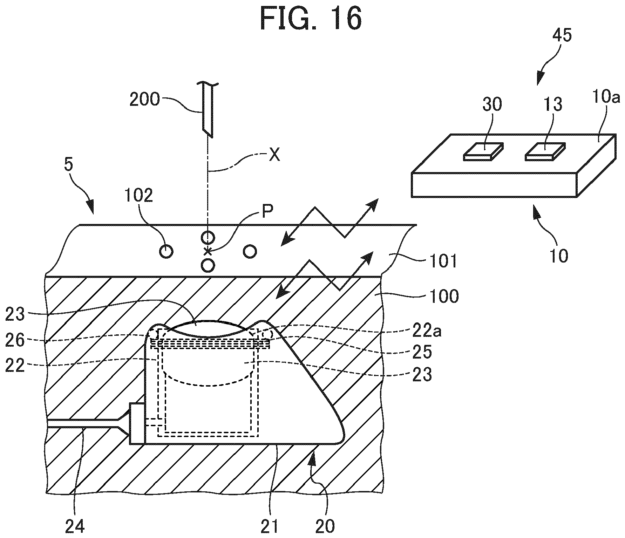

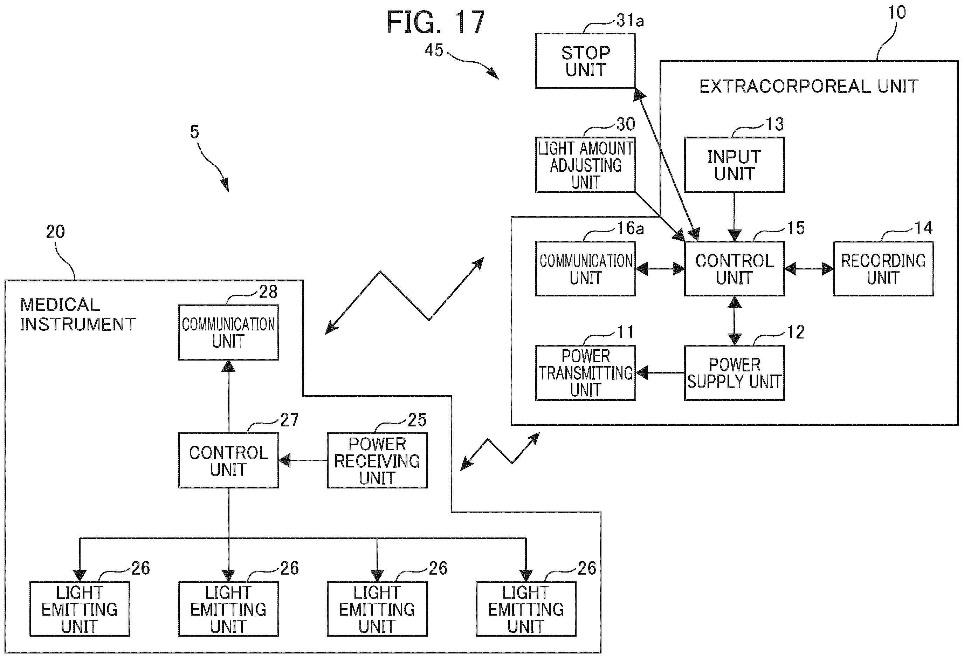

FIG. 16 is a schematic diagram showing an example of a medical device and a light amount adjusting device according to a fifth embodiment. FIG. 17 is a block diagram showing a functional configuration of the medical device and the light amount adjusting device.

A medical device 5 and a light amount adjusting device 45 of the fifth embodiment are basically the same as the medical device 1 and the light amount adjusting device 41 of the first embodiment, except that the configuration of the stop unit 31a differs from the stop unit 31, and that the communication unit 16a, the control unit 27, and the communication unit 28 are added. Therefore, herein, different configurations therebetween will be mainly described.

As shown in FIGS. 16 and 17, the medical device 5 includes an extracorporeal unit 10, a medical instrument 20, a light amount adjusting unit 30, and a stop unit 31a. The extracorporeal unit 10 further includes a communication unit 16a. In addition to the above configuration, the medical instrument 20 further includes a control unit 27 and a communication unit 28.

The control unit 27 mounted on the medical instrument 20 in the living body 100 receives the electric power received by the power receiving unit 25 and outputs the result of the magnitude of the received electric power to the communication unit 28. The communication unit 28 transmits the result of the magnitude of the electric power outputted from the control unit 27 to the communication unit 16a of the extracorporeal unit 10.

The communication unit 16a of the extracorporeal unit 10 outputs the result of the magnitude of the electric power received from the communication unit 28 of the medical instrument 20 to the control unit 15 under the control of the control unit 15. The control unit 15 activates the stop unit 31a based on the result of the magnitude of the received electric power of the power receiving unit 25 outputted from the communication unit 16a, and stops the adjustment of the light amount of the light-irradiated region by the light amount adjusting unit 30. For example, the control unit 15 refers to biometric data, the light amount data, and the data of the received electric power recorded in the recording unit 14, and stops the light amount adjusting unit 30 based on the result of the magnitude of the received electric power of the power receiving unit 25. Furthermore, the control unit 15 may autonomously learn the optimum value of the light amount of the light-irradiated region based on the results of the magnitudes of the plurality of received electric powers measured by the power receiving unit 25 and the plurality of pieces of biometric data, light amount data, and received electric power data in the recording unit 14, thereby activating the stop unit 31a. The stop of the light amount adjusting unit 30 by the stop unit 31a is automatically performed by the control unit 15.

In this way, the medical device 5 automatically activates the stop unit 31a based on the magnitude of the electric power received by the power receiving unit 25 constituting the medical instrument 20 in the living body 100. In this way, since the stop unit 31a can be automated based on the information in the living body 100 indicating the magnitude of the electric power received by the power receiving unit 25, the operation of confirming the soft portion 23 in the living body 100 becomes simpler.

The light amount adjusting device 45 includes the extracorporeal unit 10, the light amount adjusting unit 30, and the stop unit 31a. The light amount adjusting device 45 automatically activates the stop unit 31a based on the magnitude of the electric power received by the power receiving unit 25 constituting the medical instrument 20 in the living body 100. In this way, since the stop unit 31a can be automated based on the information in the living body 100 indicating the magnitude of the electric power received by the power receiving unit 25, the operation of confirming the soft portion 23 in the living body 100 becomes simpler.

According to the fifth embodiment described above, the electric power transmitted from the power transmitting unit 11 of the extracorporeal unit 10 to the medical instrument 20 in the living body 100 is received by the power receiving unit 25, and the stop unit 31a can be activated by the control unit 15 based on the result of the magnitude of the electric power received by the power receiving unit 25 in the living body 100, whereby it is possible to automate the stop unit 31a. Therefore, the operation of confirming the soft portion 23 constituting the medical instrument 20 in the living body 100 becomes simpler.

### (Sixth Embodiment)

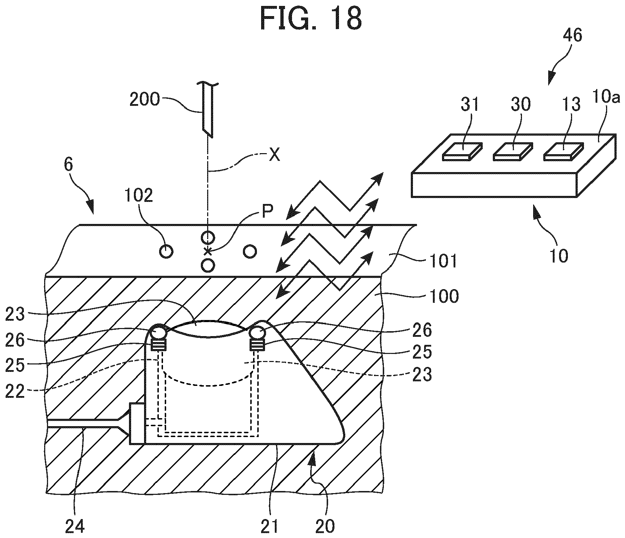

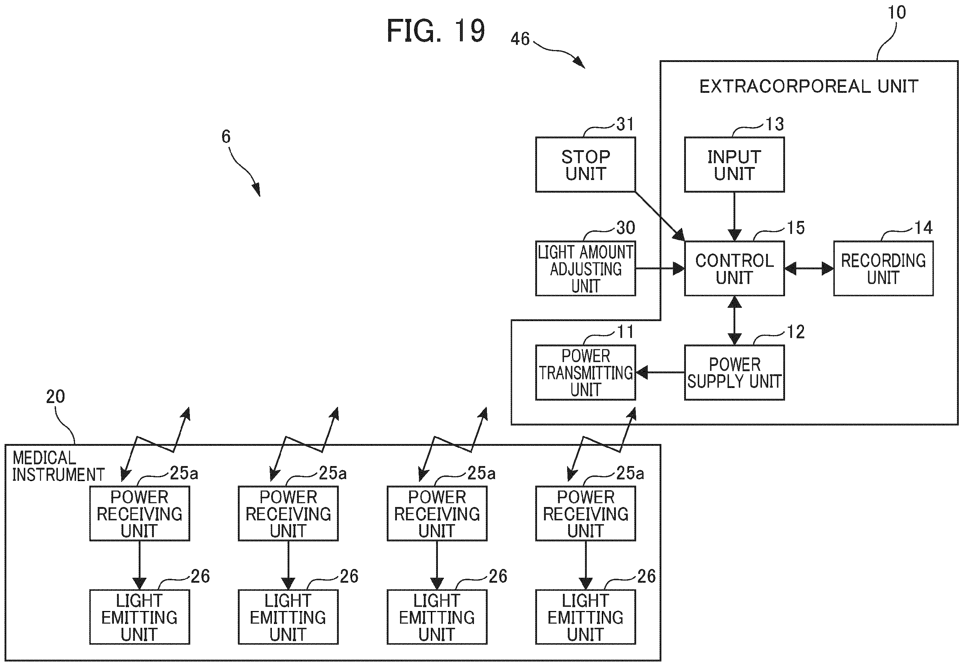

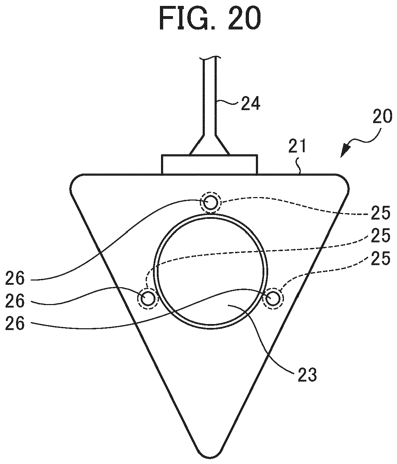

FIG. 18 is a schematic diagram showing an example of a medical device and a light amount adjusting device according to a sixth embodiment. FIG. 19 is a block diagram showing a functional configuration of the medical device and the light amount adjusting device. FIG. 20 is a schematic diagram showing an example of a medical instrument constituting the medical device.

A medical device 6 and a light amount adjusting device 46 of the sixth embodiment are basically the same as the medical device 1 and the light amount adjusting device 41 of the first embodiment except that the configuration of the power receiving unit 25a differs from the power receiving unit 25. Therefore, herein, different configurations therebetween will be mainly described.

As shown in FIGS. 18 to 20, the medical device 6 includes an extracorporeal unit 10, a medical instrument 20, and a light amount adjusting unit 30. The medical device 6 may also include a stop unit 31. The medical instrument 20 includes a power receiving unit 25a instead of the power receiving unit 25.

The medical instrument 20 includes a plurality of power receiving units 25a. Each of the power receiving units 25a is connected to a corresponding one of the light emitting units 26. The power receiving unit 25a receives electric power transmitted from the power transmitting unit 11 of the extracorporeal unit 10. For example, the power receiving unit 25a includes a coil which is wound a plurality of times around a cylindrical iron core having a diameter smaller than the diameter of the medicinal solution container 22.

Each of the light emitting units 26 emits light using electric power received by corresponding one of the connected power receiving unit 25a. The light amount of each light emitting unit 26 is determined according to the magnitude of the electric power received by a corresponding one of the power receiving units 25a. That is, the light amounts of the plurality of light emitting units 26 are each independently controlled. Furthermore, the plurality of light emitting units 26 emit light independently of each other.

The power transmitting unit 11 transmits electric power to at least one of the plurality of power receiving units 25a in a non-contact manner. For example, the user may select a desired power receiving unit 25a for transmitting the electric power when operating the input unit 13. Furthermore, when the medical device 6 includes the light amount meter 32, the control unit 15 may select a desired power receiving unit 25a for transmitting the electric power based on the result of the light amount of the light-irradiated region measured by the light amount meter 32. Furthermore, when the medical device 6 includes the control unit 27 and the communication unit 28, the control unit 15 may select a desired power receiving unit 25a for transmitting the electric power based on the result of the magnitude of the electric power received by the power receiving unit 25a of the medical instrument 20. In this way, in the medical device 6, the presence or absence of light emission of the plurality of light emitting units 26 can be individually controlled.

Furthermore, the control unit 15 adjusts the magnitude of the electric power transmitted from the power transmitting unit 11 to at least one of the plurality of power receiving units 25a by adjusting the light amount adjusting unit 30. For example, the user may select a desired power receiving unit 25a for adjusting the magnitude of the transmitted electric power when operating the light amount adjusting unit 30. Furthermore, when the medical device 6 includes the light amount meter 32, the control unit 15 may select a desired power receiving unit 25a for adjusting the magnitude of the transmitted electric power based on the result of the light amount of the light-irradiated region measured by the light amount meter 32. When the medical device 6 includes the control unit 27 and the communication unit 28, the control unit 15 may select a desired power receiving unit 25a for adjusting the magnitude of the transmitted electric power based on the result of the magnitude of the electric power received by the power receiving unit 25a of the medical instrument 20. In this way, in the medical device 6, the adjustment of the light amounts of the plurality of light emitting units 26 can be individually controlled.

Furthermore, the control unit 15 stops the adjustment of the magnitude of the electric power transmitted from the power transmitting unit 11 to at least one of the plurality of power receiving units 25a by the activation of the stop unit 31. For example, the user may select a desired power receiving unit 25a that stops adjusting the magnitude of the transmitted electric power when activating the stop unit 31. When the medical device 6 includes the light amount meter 32, the control unit 15 may select a desired power receiving unit 25a that stops adjusting the magnitude of the transmitted electric power based on the result of the light amount of the light-irradiated region measured by the light amount meter 32. When the medical device 6 includes the control unit 27 and the communication unit 28, the control unit 15 may select a desired power receiving unit 25a that stops adjusting the magnitude of the transmitted electric power based on the result of the magnitude of the electric power received by the power receiving unit 25a of the medical instrument 20. In this way, in the medical device 6, the stop of the adjustment of the light amounts of the plurality of light emitting units 26 can be individually controlled.

The light amount adjusting device 46 includes the extracorporeal unit 10 and the light amount adjusting unit 30. In the light amount adjusting device 46, the presence or absence of light emission of the plurality of light emitting units 26 and the adjustment of the light amount of the plurality of light emitting units 26 can be individually controlled. Furthermore, the light amount adjusting device 46 may include the stop unit 31. The light amount adjusting device 46 can individually control the stop of the adjustment of the light amounts of the plurality of light emitting units 26 by the stop unit 31.

According to the sixth embodiment described above, it is possible to perform control the state of the desired light-irradiated region while the plurality of light emitting units 26 are controlled individually. Therefore, it is easy to confirm the soft portion 23 constituting the medical instrument 20 in the living body 100.

While embodiments have been described above, these embodiments have been presented by way of example only, and are not intended to limit the scope of the inventions. In addition, the drawings referred to in the above description merely schematically show the shape, size, and positional relationship to the extent that the contents of the present disclosure can be understood. That is, the present disclosure is not limited to the shapes, sizes, and positional relationships illustrated in the drawings.

### EXPLANATION OF REFERENCE NUMERALS

1, 2, 3, 4, 5, 6 medical device
10 extracorporeal unit
10a housing of the extracorporeal unit
11 power transmitting unit
11a through hole
12 power supply unit
13 input unit
14 recording unit
15 control unit (extracorporeal unit-side control unit)
16, 16a communication unit (extracorporeal unit-side communication unit)
17 power transmitting coil
20 medical instrument
21 main body portion
22 medicinal solution container
22a opening
23 soft portion
24 catheter
25, 25a power receiving unit
26 light emitting unit
27 control unit (medical instrument-side control unit)
28 communication unit (medical instrument-side communication unit)
30, 30a light amount adjusting unit
31, 31a stop unit
32 light amount meter
41, 42, 43, 44, 45, 46 light amount adjusting device
100 living body
101 body surface
102 light-irradiated portion
200 injection needle
P puncture target area

## Claims

1. A medical device comprising:
an extracorporeal unit including a power transmitting unit that transmits electric power from outside of a living body into the living body in a non-contact manner;
a medical instrument embedded in the living body, the medical instrument including a power receiving unit that receives the electric power transmitted from the power transmitting unit, a soft portion through which an injection needle is inserted, and a plurality of light emitting units that are provided along an outer edge of the soft portion and are each configured to emit light using the electric power received by the power receiving unit; and
a light amount adjusting unit that adjusts a light amount of a light-irradiated region including one or more light-irradiated portions to be projected on a body surface from the plurality of light emitting units.

2. The medical device according to claim 1, wherein the light amount adjusting unit adjusts a light amount of the light-irradiated region so that a center of the light-irradiated region is darkened.

3. The medical device according to claim 1 or 2, wherein the light amount adjusting unit performs adjusting by weakening the light amount of the light-irradiated region.

4. The medical device according to any one of claims 1 to 3, wherein the light amount adjusting unit simultaneously adjusts light amounts of the plurality of light emitting units.

5. The medical device according to any one of claims 1 to 4, further comprising a stop unit that stops the light amount adjusting unit.

6. A light amount adjusting device comprising:
an extracorporeal unit including a power transmitting unit that transmits electric power from outside of a living body to a medical instrument embedded in the living body in a non-contact manner, the medical instrument including a plurality of light emitting units that are each configured to emit light, and
a light amount adjusting unit that adjusts a light amount of a light-irradiated region including one or more light-irradiated portions to be projected on a body surface from the plurality of light emitting units.
